# EUROPEAN PATENT SPECIFICATION

(11) **EP 4 580 491 B1**
(45) Date of publication and mention of the grant of the patent: **30.09.2026**
(21) Application number: 23761816.0
(22) Date of filing: 23.08.2023
(51) Int. Cl.: A61B 5/0531, A61B 5/00, A61B 5/053

(54) **BIOIMPEDANCE MEASUREMENT CIRCUIT, METHOD AND ELECTRONIC DEVICE**
SCHALTUNG, VERFAHREN UND ELEKTRONISCHES GERÄT ZUR BIOIMPEDANZMESSUNG
CIRCUIT DE MESURE DE LA BIOIMPÉDANCE, MÉTHODE ET DISPOSITIF ÉLECTRONIQUE

(30) Priority: 30.08.2022 DE 102022121888; 22.11.2022 DE 102022130841
(43) Date of publication of application: 09.07.2025
(73) Proprietor: ams Sensors Germany GmbH, 07745 Jena (DE)
(72) Inventor: SALDANA, Julio, 07745 Jena (DE); SASIKALA JAYACHANDRAN PILLAI, Parvathy, 99085 Erfurt (DE); MICAKOVIC, Predrag, 99084 Erfurt (DE)
(74) Representative: Epping - Hermann - Fischer
(86) International application number: PCT/EP2023/073091
(87) International publication number: WO 2024/046827

(56) References cited:
- US-A1- 2020 096 463
- US-A1- 2022 071 503

## Description

### Technical Field

The disclosure relates to a bioimpedance measurement circuit for measuring a bioimpedance across measurement electrodes, and to an electronic device with such bioimpedance measurement circuit. The disclosure further relates to a corresponding bioimpedance measurement method and to a computer program product therof.

### Related applications

This patent application claims the priority of German patent applications 102022121888.0 and 102022130841. 3.

### Background

Bioimpedance measurement is a biomedical technique to determine the electrical behavior of living tissue by using an electrode arrangement for applying a stimulus current to a sample, for example a part of a human body, and measure the resulting voltage through a four point measurement method. The amplitude and phase of the resulting voltage signal will depend on the BIOZ impedance. The bioimpedance of interest may be determined by the measured voltage divided by the stimulus current injected into the tissue.

Due to asymmetries in a current path of the arrangement of the measuring electrodes to perform the four point measurement method, a common mode signal is created across the body impedance. Those asymmetries arise because of a mismatch between the skin/electrode contact impedances. Moreover, due to asymmetries in the voltage path between the measuring electrodes and a differential amplifier to evaluate the voltage across the measuring electrodes, the common mode signal is translated to an extra differential signal that is added to the desired differential signal. As a result, the measured magnitude of the total input differential signal which is applied to the differential amplifier is higher than the expected one and the phase is also affected.

Document US 2022/071503 A1 discloses a bioimpedance measurement circuit configured to compensate for a leakage of an electric signal resulting from a parasitic component during bioimpedance measurement. The circuit comprises four electrodes and respective circuitry for connecting and measuring currents and voltages at the four electrodes.

Similarly, document US 2020/096463 A1 discloses a bioimpedance measurement configured to allow the unknown bioimpedance and contact impedances to be derived.

### Summary

An object to be achieved is to provide an improved bioimpedance measurement concept which provides the bioimpedance of interest as accurately as possible, and in particular attenuates effects of mismatch between the skin/electrode contact impedances.

This object is achieved with the subject-matter of the independent claims. Embodiments and developments derive from the dependent claims.

Bioimpedance measurement usually is performed by applying a current to the sample and measure the generated voltage through a 4 point measurement method. The applied current is an alternating current, for example a 50Khz sine wave.

The amplitude and phase of the resulting voltage signal will depend on the bioimpedance or, in general, body impedance.

The improved bioimpedance measurement concept is based on the finding that the actual measurement of the body impedance is affected by errors due to unavoidable mismatch in contact impedances resulting in common mode signals. Hence a correction is proposed that is based on various contact impedances being effective during application of the four electrodes onto the body. However, since even these impedances are subject to parasitic impedances, a correction of the measured body impedance to determine a corrected body impedance needs to be based on various impedances of body parts and corresponding stored parasitic impedances, which may be determined during a calibration phase. Hence for each measurement of a body impedance at least two bodypart impedance measurements have to be performed, the bodyparts e.g. corresponding to a location, where the electrodes are applied, like a wrist and a finger, in case of e.g. usage in a wristband or watch on a wrist.

In an embodiment of a bioimpedance measurement circuit according to the improved bioimpedance measurement concept the bioimpedance measurement circuit comprises a set of terminals comprising a first terminal for connecting a first electrode, a second terminal for connecting a second electrode, a third terminal for connecting a third electrode and a fourth terminal for connecting a fourth electrode, each electrode to be attached to a body. A control circuit is included to control application of a stimulus current with a measurement frequency, in particular an AC current, through a first subset of two selected terminals of the set of terminals and measurement of an input voltage in response to the stimulus current at a second subset of two selected terminals of the set of terminals. An evaluation circuit is included for determining a measured impedance in response to the stimulus current and the measured input voltage. A signal processing circuit determines a body impedance.

The control circuit is configured to control measurement of a body impedance, where the stimulus current is applied through the first and the second terminal, and the input voltage is measured between the third and the fourth terminal, of a first bodypart impedance, where the stimulus current is applied through the first and the third terminal, and the input voltage is measured between the first and the third terminal, and of a second bodypart impedance, where the stimulus current is applied through the second and the fourth terminal, and the input voltage is measured between the second and the fourth terminal. The signal processing circuit is configured to determine an error impedance based on the measured first bodypart impedance, the measured second bodypart impedance, a stored first parasitic bodypart impedance, a stored second parasitic bodypart impedance, and a stored input impedance, and to determine a corrected body impedance based on the measured body impedance and the error impedance.

Hence with only two additional measurements, i.e. of the bodypart impedances, a more accurate result of the body impedance can be achieved.

According to the invention, the signal processing circuit is configured to determine the corrected body impedance as a difference between the measured body impedance and the error impedance.

According to the invention, the signal processing circuit is further configured to determine a first corrected bodypart impedance Zwc as a parallel equivalent of the measured first bodypart impedance and the stored first parasitic bodypart impedance, to determine a second corrected bodypart impedance Zfc as a parallel equivalent of the measured second bodypart impedance and the stored second parasitic bodypart impedance, and to determine the error impedance Zerr based on the first corrected bodypart impedance Zwc, the second corrected bodypart impedance Zfc and the stored input impedance Zin. This is implemented with the following equation: $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$

In some implementations the control circuit is further configured to control measurement of a total impedance, where the stimulus current is applied through the third and the fourth terminal, and the input voltage is measured between the third and the fourth terminal. In such implementation the signal processing circuit may be further configured to determine a corrected total impedance as a parallel equivalent of the measured total impedance and a stored parasitic total impedance, and to determine the corrected body impedance further based on the corrected total impedance.

In some implementations the signal processing circuit is further configured to determine the stored first parasitic bodypart impedance, the stored second parasitic bodypart impedance and the stored input impedance during a calibration phase.

For example, during the calibration phase the first, second third and fourth terminals are connected to a calibration body via respective electrodes. The signal processing circuit is configured to determine the stored first parasitic bodypart impedance, the stored second parasitic bodypart impedance and the stored input impedance based on respective measurements and known impedance values of the calibration body corresponding to the measurements. Such calibration may be performed during or at the end of a production phase, such that the corresponding parasitic impedances and the input impedance can be stored in a memory of the bioimpedance measurement circuit.

The electrode arrangement including the first electrode, the second electrode, the third electrode and the fourth electrode respectively connected to the corresponding terminal of the set of terminals may be part of the bioimpedance measurement circuit.

In an embodiment of a bioimpedance measurement method according to the improved bioimpedance measurement concept the bioimpedance measurement method is performed with a set of terminals comprising a first terminal connected to a first electrode being attached to a body, a second terminal connected to a second electrode being attached to the body, a third terminal connected to a third electrode being attached to the body, and a fourth terminal connected to a fourth electrode being attached to the body. The method comprises:
- generating a stimulus current with a measurement frequency;
- determining a body impedance in response to the stimulus current and a measured input voltage in response to the stimulus current, where the stimulus current is applied through the first and the second terminal, and the input voltage is measured between the third and the fourth terminal;
- determining a first bodypart impedance in response to the stimulus current and the measured input voltage, where the stimulus current is applied through the first and the third terminal, and the input voltage is measured between the first and the third terminal;
- determining a second bodypart impedance in response to the stimulus current and the measured input voltage, where the stimulus current is applied through the second and the fourth terminal, and the input voltage is measured between the second and the fourth terminal;
- determining a first corrected bodypart impedance Zwc as a parallel equivalent of the measured first bodypart impedance Zw and a stored first parasitic bodypart impedance;
- determining a second corrected bodypart impedance Zfc as a parallel equivalent of the measured second bodypart impedance Zf and a stored second parasitic bodypart impedance;
- determining an error impedance Zerr according to $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ wherein Zin is a stored input impedance; and
- determining a corrected body impedance as a difference between the measured body impedance and the error impedance Zerr.

The bioimpedance measurement method may be a computer implemented method that e.g. is carried out by a processor or programmable circuit.

Further implementations of the method become readily apparent for the skilled reader from the various implementations described above in conjunction with the bioimpedance measurement circuit.

According to one embodiment of the improved bioimpedance measurement concept, a computer program product is disclosed, the computer program product comprising instructions which, when executed on one or more processors in connection with a first, a second, a third and a fourth terminal, e.g. as described above, cause the one or more processors to perform the bioimpedance measurement method according to one of the disclosed implementations.

The various implementations of the bioimpedance measurement circuit and method may be used in various applications and products, e.g. electronic devices, like vital sign monitoring in wearables (smartwatches) or healthcare applications like diagnostics. The body impedance can for example be the basis for determination of Body-Cell-Mass Composition (BCM), hydration level detection, calories consumption, fat, muscle percentage, stress level, to name only a few.

### BRIEF DESCRIPTION OF DRAWINGS

The improved bioimpedance measurement concept will be explained in more detail in the following with the aid of the drawings. Elements and functional blocks having the same or similar function bear the same reference numerals throughout the drawings. Hence their description is not necessarily repeated in the following drawings.

In the drawings:
- Figure 1: shows an example implementation of a bioimpedance measurement circuit with asymmetries in the current/voltage paths because of mismatch between the skin/electrode contact impedances;
- Figure 2: shows a further example implementation of a bioimpedance measurement circuit;
- Figure 3: shows a detail of the bioimpedance measurement circuit of Figure 2;
- Figure 4: shows various measurement configurations for a calibration phase of a bioimpedance measurement circuit;
- Figure 5: shows various measurement configurations for a measurement phase of a bioimpedance measurement circuit;
- Figure 6: shows a detail of the bioimpedance measurement circuit used in Fig. 5 and Fig. 6;
- Figure 7: shows an example block diagram of operational phases in a bioimpedance measurement circuit;
- Figure 8: shows another example block diagram of operational phases in a bioimpedance measurement circuit; and
- Figure 9: shows another example implementation of a bioimpedance measurement circuit.

### DETAILED DESCRIPTION

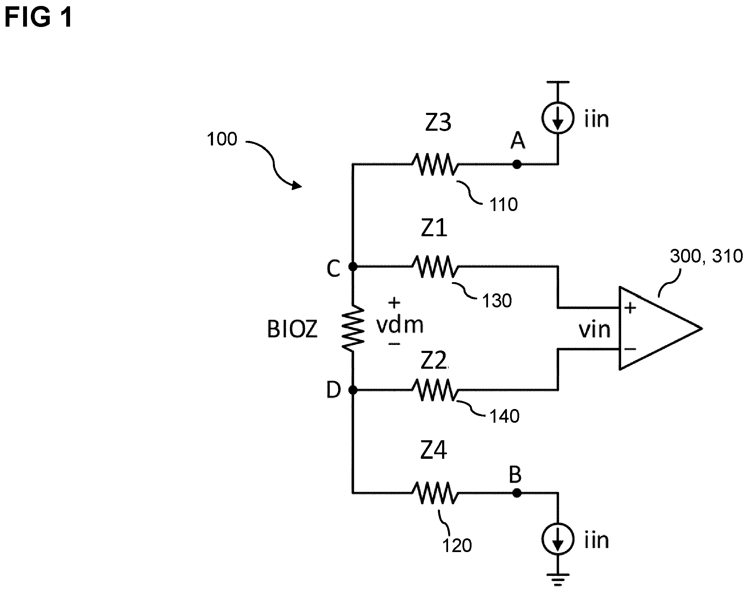

Figure 1 illustrates a bioimpedance measurement circuit that is based on a four point measurement method. The bioimpedance measurement circuit comprises an electrode arrangement 100 for attaching to a human body. The electrode arrangement 100 includes a first pair of electrodes 110, 120 to apply a stimulus current iin. The electrodes 110, 120 are attached to nodes A and B of a human body. It is assumed that electrode 110 has an electrode contact impedance Z3, and electrode 120 has an electrode contact impedance Z4. The electrode arrangement further comprises a second pair of electrodes 130, 140 to measure a voltage vin between the electrodes 130, 140 of the second pair. The electrodes 130, 140 are attached to nodes C and D of a human body. It is assumed that electrode 130 has an electrode contact impedance Z1, and electrode 140 has an electrode contact impedance Z2.

The electrodes 110, 120, 130 and 140 are placed to measure a bioimpedance BIOZ of interest between nodes C and D of the human body or between electrodes 130 and 140. The generated voltage vin is applied to and evaluated by an evaluation circuit 300 which is illustrated in simplified form as a differential amplifier 310 in Figure 1. The amplitude and phase of the resulting voltage signal vin depends on the bioimpedance BIOZ of interest, and the bioimpedance BIOZ is the quotient vin/iin.

Ideally, the voltage vin at the input of the evaluation circuit 300 is equal to the desired differential signal vdm which represents the voltage drop across the bioimpedance BIOZ. In reality, however, due to asymmetries in the current path a common mode signal is created across the body impedance BIOZ. Assuming that in some implementations, the common mode at nodes A and B is regulated to be some internal defined reference voltage vref, the common mode signal across nodes C and D is: $\frac{VC + VD}{2} = \frac{VA + VB}{2} + \frac{Z 4 - Z 3}{2} ⋅ iin = vref + k ⋅ iin$ where VA is the voltage potential at node A, VB is the voltage potential at node B, VC is the voltage potential at node C, and VD is the voltage potential at node D.

As a result, a common mode signal vcm is generated across the bioimpedance BIOZ between nodes C and D, wherein the common mode signal vcm is proportional to the stimulus current: $vcm = \frac{Z 4 - Z 3}{2} ⋅ iin$

Due to asymmetries in the voltage path, the common mode signal vcm is translated to an unwanted extra differential signal vdmx. Those asymmetries arise because of mismatch between the skin/electrode contact impedances.

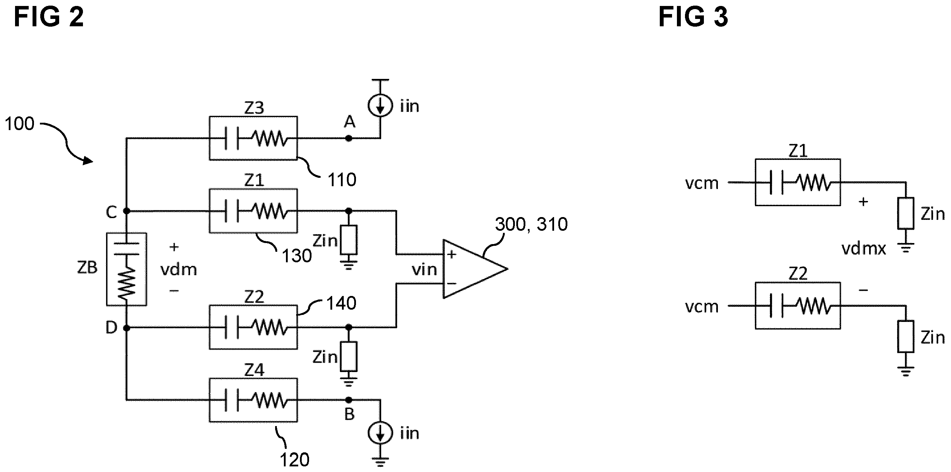

Figure 2 shows a further example implementation of a bioimpedance measurement circuit, which is similar to the implementation of Figure 1 and includes input impedances Zin at the inputs of amplifier 310. Furthermore, it can be seen that the impedances at the electrodes 110, 120, 130, 140 have both a resistive and a capacitive component.

Figure 3 shows a detail of the bioimpedance measurement circuit of Figure 2. Let Zin be the single ended input impedance of the amplifier 310. It is expected that this impedance is mainly capacitive, but in general it can be modeled as a parallel combination of a resistor and a capacitor: $Zin = \frac{Rin}{1 + j ⋅ ω ⋅ Rin ⋅ Cin}$

Due to the mismatch of the contact impedances, the common mode signal in the body will be translated to a differential signal vdmx at the input of the amplifier 310: $vdmx = \frac{Zin}{Z 1 + Zin} ⋅ vcm - \frac{Zin}{Z 2 + Zin} ⋅ vcm$

Using the expression for vcm obtained above vdmx results to: $vdmx = \left(\frac{Zin ⋅ \left(Z2-Z1\right)}{\left(Z1+Zin\right) ⋅ \left(Z2+Zin\right)}\right) ⋅ \left(\frac{Z 4 - Z 3}{2}\right) ⋅ iin$

Referring back to Figure 2, let Zb be the body impedance, then assuming for now no significant attenuation in the differential path, the expected measured differential voltage vdm is: $vdm = iin ⋅ Zb$

However, due to the error of the common mode signal the total input differential voltage will be: $vin = vdm + vdmx ,$ or $vin = iin ⋅ Zb + \left(\frac{Zin ⋅ \left(Z2-Z1\right)}{\left(Z1+Zin\right) ⋅ \left(Z2+Zin\right)}\right) ⋅ \left(\frac{Z 4 - Z 3}{2}\right) ⋅ iin$

Then the measured (body) impedance will be: $Zmeas = \frac{vin}{iin} = Zb + \left(\frac{Zin ⋅ \left(Z2-Z1\right)}{\left(Z1+Zin\right) ⋅ \left(Z2+Zin\right)}\right) ⋅ \left(\frac{Z 4 - Z 3}{2}\right) = Zb + Zerr$

The final expression for the error impedance Zerr is then: $Zerr = \left(\frac{Zin ⋅ \left(Z2-Z1\right)}{\left(Z1+Zin\right) ⋅ \left(Z2+Zin\right)}\right) ⋅ \left(\frac{Z 4 - Z 3}{2}\right)$

Assuming that the contact impedances at both inputs of the amplifier 310 are equal, i.e. for a first bodypart impedance, e.g. a wrist impedance Zw, and a second bodypart impedance, e.g. a finger impedance Zf, $Z 3 \approx Z 1 = Zw$ and $Z 4 \approx Z 2 = Zf ,$ the error impedance expression for Zerr becomes: $Zerr = \frac{1}{2} ⋅ \left(\frac{Zin ⋅ {\left(Zf-Zw\right)}^{2}}{\left(Zw+Zin\right) ⋅ \left(Zf+Zin\right)}\right)$

Hence the corrected body impedance results as a difference between the measured body impedance Zmeas and the error impedance Zerr.

For example, the correction method according to the improved bioimpedance measurement concept includes estimating by measurement and calibration, the values of Zf, Zw and Zin, calculate the error impedance Zerr and subtract it from the measured body impedance.

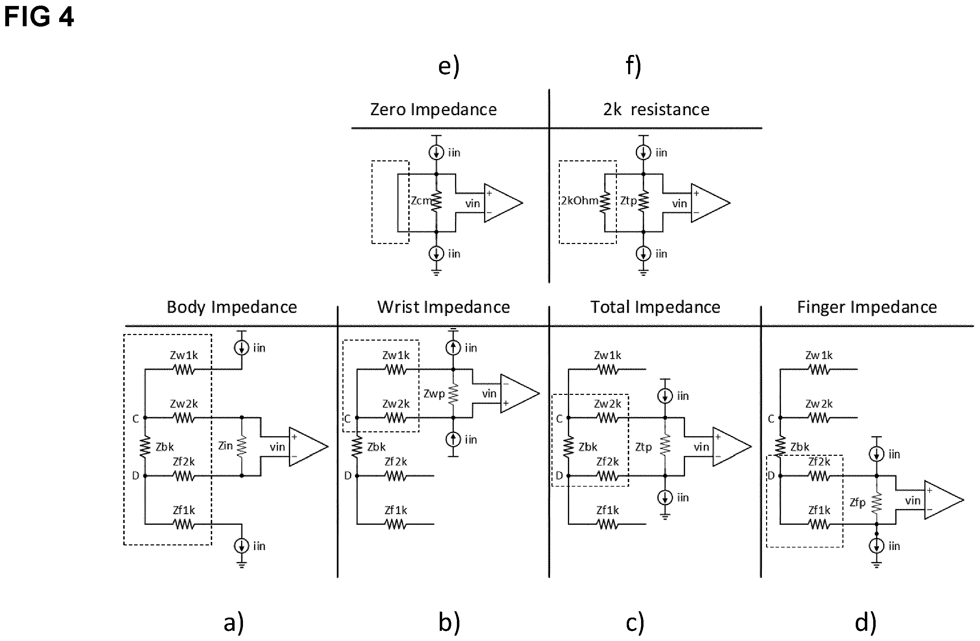

Figure 4 shows various measurement configurations a) to f) for a calibration phase of a bioimpedance measurement circuit. As described, there is a dependence of the error impedance Zerr on the first bodypart impedance or wrist impedance Zw, on the second bodypart impedance or finger impedance Zf and on the input impedance Zin, the values of which have to be known for each measurement of the body impedance. While the input impedance Zin of the amplifier can be assumed to remain the same, the effective bodypart impedances may change with e.g. changing application of the electrodes.

For example, in configuration a) for measuring a body impedance, the stimulus current iin is applied through the first and the second terminal, and the input voltage vin is measured between the third and the fourth terminal or the corresponding electrodes, respectively. Similarly, in configuration b) for measuring a first bodypart impedance or wrist impedance Zw, the stimulus current iin is applied through the first and the third terminal, and the input voltage vin is measured between the first and the third terminal. In configuration c) a total impedance Zt can be measured, where the stimulus current iin is applied through the third and the fourth terminal, and the input voltage vin is measured between the third and the fourth terminal. Similarly, in configuration d) for measuring a second bodypart impedance or finger impedance Zf, the stimulus current iin is applied through the second and the fourth terminal, and the input voltage vin is measured between the second and the fourth terminal or the corresponding electrodes, respectively.

During the calibration phase, the electrodes are placed on a body or other object with known impedances like a known body impedance Zbk, known wrist impedances Zwlk, Zw2k and known finger impedances Zf1k, Zf2k.

In configurations e) and f) a zero impedance and a known resistance impedance like 2kΩ are measured.

For example, ideally, with the configuration b) of Figure 4 it is possible to measure directly 2*Zw. However when the magnitude of 2*Zw is high enough, the current leakage through a parasitic parallel path can cause significant error. In order to correct this error it is proposed to have a calibration phase, where the impedance Zwp of the parallel parasitic path is estimated.

During the calibration phase a known reference impedance is used, so we can know in advance the value of the impedance.

Let Zwlk, Zw2k be the known wrist impedances of the selected reference impedance for calibration. So, due to the parasitic parallel path, instead of measuring Zw1k+Zw2k the system will measure a different value that we will denote as Zwm.

The measured contact impedance will be $Zwm = \left.\left(Zw1k+Zw2k\right)\right‖ Zwp$

By using known impedances Zwlk, Zw2k, we can calculate the parasitic impedance by solving the equation to: $Zwp = \frac{Zwm \left(Zw1k+Zw2k\right)}{Zw 1 k + Zw 2 k - Zwm}$

Similar approaches for the other configurations will readily be apparent to the skilled reader

In general, during calibration the known reference impedances are connected, and four values are measured:
- Body impedance Zb
- Wrist impedance Zw (divide the result by 2 to get Zw)
- Finger impedance Zf (divide the result by 2 to get Zf)
- Total impedance Zt(optional)

The known values of the reference impedances will be denoted as:
- Known body impedance: Zbk
- Known wrist impedance: Zwlk, Zwk2
- Known finger impedance: Zflk, Zf2k
- Known total impedance: Ztk=Zbk+Zw2k+Zf2k

Based on the previous information, 4 values are calculated
- Equivalent impedance in parallel with wrist: Zwp
- Equivalent impedance in parallel with finger: Zfp
- Equivalent impedance in parallel with total: Ztp (optional)
- Equivalent single ended input impedance: Zin

Equations e.g. used during calibration:
Intermediate parameter fac (Current division factor for total impedance): $fac = \frac{Ztp}{Ztp + Ztk}$

Intermediate parameter Zberr (Body impedance error): $Zberr = \frac{Zb}{fac} - Zbk$

For determining the input impedance Zin the quadratic equation of the error impedance Zerr is resolved: $a = 2 ⋅ Zberr$ $b = 2 ⋅ Zberr ⋅ \left(Zfk+Zwk\right) - {\left(Zfk-Zwk\right)}^{2}$ $c = 2 ⋅ Zberr ⋅ Zfk ⋅ Zwk$ $delta = {b}^{2} - 4 ⋅ a ⋅ c$ $Zin = \frac{- b + \sqrt{delta}}{2 ⋅ a}$

If imaginary part of Zin is positive then $Zin = \frac{- b - \sqrt{delta}}{2 ⋅ a}$

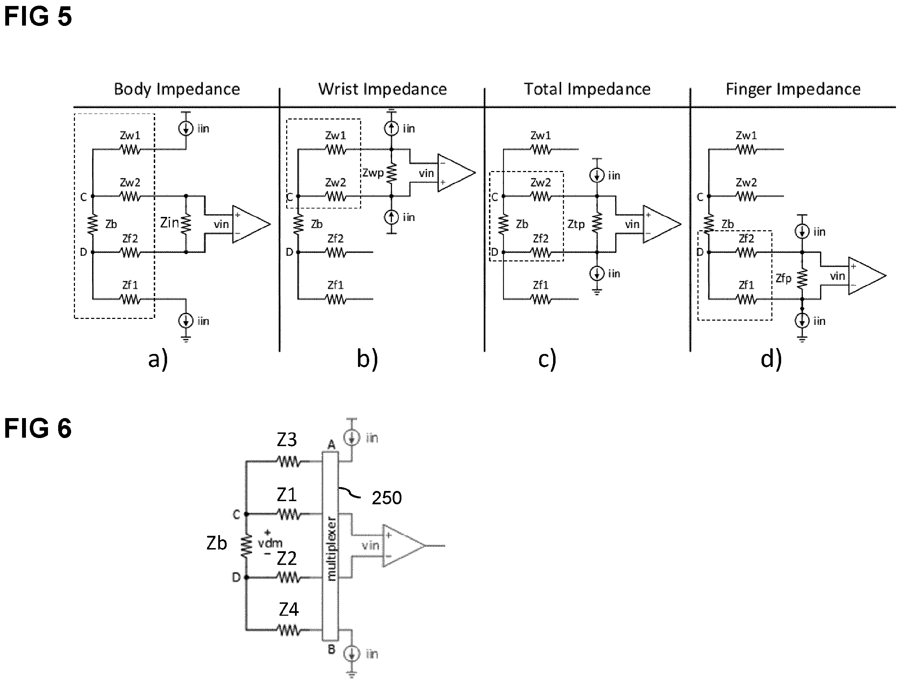

Referring now to Figure 5, various measurement configurations for a measurement phase of a bioimpedance measurement circuit are shown. The respective configurations a) to d), which for example can be achieved employing a multiplexer 250 as shown in Fig. 6, correspond to those described for Figure 4. The only difference is that the respective impedances are not known. However, at this stage the parasitic impedances are available for performing the corrections.

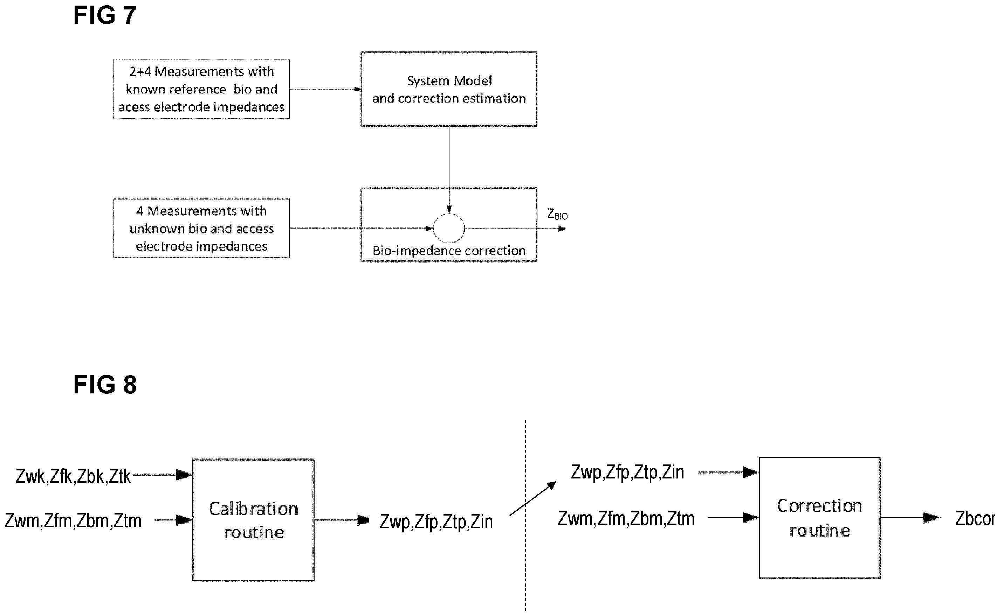

Figure 7 shows an example block diagram of operational phases in a bioimpedance measurement circuit. For example, the system model and correction estimation corresponds to the calibration phase having as its input the 2+4 measurements and the known impedances, as described above. During the regular operation or measurement operation, only the four measurements of Figure 5 are made, which form the basis for the actual determination of the body impedance, as also described above.

As shown in Figure 8, the calibration routine receives the measured values Zw, Zf, Zt, Zb, wherein Zt may be optional, for the known body, together with the know impedances Zwk, Zfk, Zbk, Ztk. As described, the result is the parasitic impedances Zwp, Zfp, Ztp and the input impedance Zin. During the regular operation or measurement operation the correction routine (also) receives the measured values Zw, Zf, Zt, Zb, wherein Zt may be optional, and performs the respective corrections based on the parasitic impedances Zwp, Zfp, Ztp and the input impedance Zin in order to determine the corrected body impedance Zb.

Equations used during measurement and correction:
Let's denote the measured values as Zwm, Zfm, Zbm, Ztm. Let's denote corrected values as Zwc, Zfc, Zbc, Ztc.

### Corrected impedances:

$Zwc = Zwm \left‖\left(-Zwp\right)\right.$$Zfc = Zfm \left‖\left(-Zfp\right)\right.$$Ztc = Ztm \left‖\left(-Ztp\right)\right.$

The corrected impedances Zwc and Zfc, together with Zin, need to be used for calculating the error impedance Zerr as disclosed above, for each measurement.

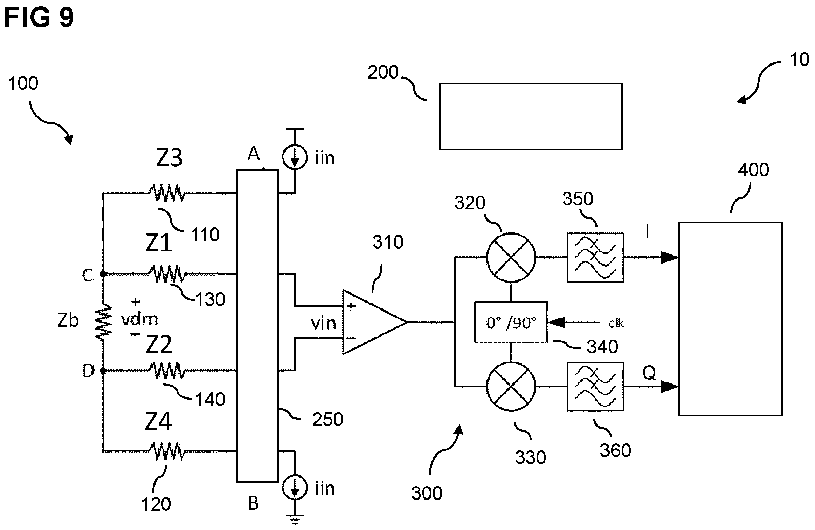

A bioimpedance measurement circuit 10 which allows to determine the real part I and the imaginary part Q of a measured bioimpedance is shown in Figure 9. The measurement circuit 10 comprises an electrode arrangement 100 for attaching to a body. The electrode arrangement 100 includes four electrodes 110, 120, 130, 140 to apply a stimulus current iin, and to measure an input voltage vin, depending on a configuration selected via multiplexer 250. The electrodes 110, 120 are attached to nodes A and B of a human body, wherein Z3 denotes an electrode contact impedance between the skin of the body and the electrode 110, and Z4 denotes an electrode contact impedance between the skin of the body and the electrode 120.

The bioimpedance measurement circuit 10 further comprises a control circuit 200 to control respective impedance measurements, and an evaluation circuit 300. The evaluation circuit 300 is configured for determining a real part I and an imaginary part Q of the measured impedance in response to the stimulus current iin and the measured input voltage vin.

The evaluation circuit 300 comprises a differential amplifier 310 to apply the measured input voltage vin. An output side of the differential amplifier 310 is coupled to a first path comprising a modulator 320 and a low pass filter 350, and a second path comprising a modulator 330 and a low pass filter 360. The modulators 320 and 330 are coupled to an oscillator 340. The arrangement of modulators 320, 330 coupled to oscillator 340 with low pass filters 350, 360 arranged behind the modulators 320, 330 is provided to implement a quadrature demodulation which allows to measure the real part I and the imaginary part Q of the measured impedance.

Referring to Figure 9, the bioimpedance measurement circuit comprises a signal processing circuit 400, e.g. a processor for determining a corrected value of the bioimpedance (BIOZ), i.e. for performing the corresponding calculations as described above. To this end the signal processing circuit 400 may comprise or be connected to a memory for storing the values needed for correction.

The control circuit 200 is configured to control the measurement of the impedances by applying the stimulus current iin with a measurement frequency F to the electrode arrangement 100, in particular by controlling the multiplexer 250 according to the desired impedance.

The control circuit 200 may also control the different operating modes, i.e. calibration operation and regular operation or measurement operation, respectively. The control circuit 200 itself may be controlled by the signal processing circuit 400.

The embodiments of the improved bioimpedance measurement concept disclosed herein have been discussed for the purpose of familiarizing the reader with novel aspects of the implementation of the improved bioimpedance measurement concept. Although preferred embodiments have been shown and described, many changes, modifications, equivalents and substitutions of the disclosed concepts may be made by one having skill in the art without departing from the scope of the claims.

In particular, the implementation of the improved bioimpedance measurement concept is not limited to the disclosed embodiments, and gives examples of many alternatives possible for the features included in the embodiments discussed. However, it is intended that any modifications, equivalents and substitutions of the disclosed concepts be included within the scope of the claims which are appended hereto.

Features recited in separate dependent claims may be advantageously combined. Moreover, reference signs used in the claims are not limited to be construed as limiting the scope of the claims.

Furthermore, as used herein, the term "comprising" does not exclude other elements. In addition, as used herein, the article "a" is intended to include one or more than one component or element, and is not limited to be construed as meaning only one.

### References

- 10: bioimpedance measurement circuit
- 100: electrode arrangement
- 110, 120, 130, 140: electrodes
- 200: control circuit
- 250: multiplexer
- 300: evaluation circuit
- 310: differential amplifier
- 320: modulator
- 330: modulator
- 340: oscillator
- 350: low pass filter
- 360: low pass filter
- 400: signal processing circuit
- Z1, Z2, Z3, Z4: electrode contact impedance
- BIOZ, Zb: bioimpedance
- I: real part
- Q: imaginary part

## Claims

1. A bioimpedance measurement circuit, comprising
- a set of terminals comprising
- a first terminal for connecting a first electrode (110) to be attached to a body;
- a second terminal for connecting a second electrode (120) to be attached to the body;
- a third terminal for connecting a third electrode (130) to be attached to the body;
- a fourth terminal for connecting a fourth electrode (140) to be attached to the body;
- a control circuit (200) to control application of a stimulus current (iin) with a measurement frequency (F) through a first subset of two selected terminals of the set of terminals and measurement of an input voltage (vin) in response to the stimulus current (iin) at a second subset of two selected terminals of the set of terminals;
- an evaluation circuit (300) for determining a measured impedance in response to the stimulus current (iin) and the measured input voltage (vin); and
- a signal processing circuit (400) for determining a body impedance (Zb);
**characterized in that**
the control circuit (200) is configured to control measurement of
- a body impedance (Zb), where the stimulus current (iin) is applied through the first and the second terminal, and the input voltage (vin) is measured between the third and the fourth terminal;
- a first bodypart impedance Zw, where the stimulus current (iin) is applied through the first and the third terminal, and the input voltage (vin) is measured between the first and the third terminal; and
- a second bodypart impedance Zf, where the stimulus current (iin) is applied through the second and the fourth terminal, and the input voltage (vin) is measured between the second and the fourth terminal; and that
the signal processing circuit (400) is configured to
- determine a first corrected bodypart impedance Zwc as a parallel equivalent of the measured first bodypart impedance Zw and a stored first parasitic bodypart impedance (Zwp);
- determine a second corrected bodypart impedance Zfc as a parallel equivalent of the measured second bodypart impedance Zf and a stored second parasitic bodypart impedance (Zfp);
- determine an error impedance Zerr according to $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ wherein Zin is a stored input impedance; and
- determine a corrected body impedance (Zbcorr) as a difference between the measured body impedance (Zb) and the error impedance Zerr.

2. The bioimpedance measurement circuit according to claim 1 to, wherein the control circuit (200) is further configured to control measurement of a total impedance (Zt), where the stimulus current (iin) is applied through the third and the fourth terminal, and the input voltage (vin) is measured between the third and the fourth terminal, and wherein the signal processing circuit (400) is further configured to determine a corrected total impedance (Ztc) as a parallel equivalent of the measured total impedance (Zt) and a stored parasitic total impedance (Ztp), and to determine the corrected body impedance (Zbcorr) further based on the corrected total impedance (Ztc).

3. The bioimpedance measurement circuit according to one of claims 1 or 2, wherein the signal processing circuit (400) is further configured to determine the stored first parasitic bodypart impedance Zwp, the stored second parasitic bodypart impedance Zf and the stored input impedance Zin during a calibration phase.

4. The bioimpedance measurement circuit according to claim 3, wherein during the calibration phase the first, second third and fourth terminals are connected to a calibration body via respective electrodes, and wherein the signal processing circuit (400) is configured to determine the stored first parasitic bodypart impedance (Zwp), the stored second parasitic bodypart impedance (Zfp) and the stored input impedance Zin based on respective measurements and known impedance values of the calibration body corresponding to the measurements.

5. The bioimpedance measurement circuit according to one of claims 1 to 4, further comprising an electrode arrangement (100) for attaching to a body, the electrode arrangement (100) including the first electrode (110), the second electrode (120), the third electrode (130) and the fourth electrode (140) respectively connected to the corresponding terminal of the set of terminals.

6. An electronic device comprising a bioimpedance measurement circuit according to one of claims 1 to 5.

7. A bioimpedance measurement method being performed with a set of terminals comprising
- a first terminal connected to a first electrode (110) being attached to a body;
- a second terminal connected to a second electrode (120) being attached to the body;
- a third terminal connected to a third electrode (130) being attached to the body; and
- a fourth terminal connected to a fourth electrode (140) being attached to the body;
the method comprising:
- generating a stimulus current (iin) with a measurement frequency (F);
**characterized in that** the method further comprises
- determining a body impedance (Zb) in response to the stimulus current (iin) and a measured input voltage (vin) in response to the stimulus current (iin), where the stimulus current (iin) is applied through the first and the second terminal, and the input voltage (vin) is measured between the third and the fourth terminal;
- determining a first bodypart impedance Zw in response to the stimulus current (iin) and the measured input voltage (vin), where the stimulus current (iin) is applied through the first and the third terminal, and the input voltage (vin) is measured between the first and the third terminal;
- determining a second bodypart impedance Zf in response to the stimulus current (iin) and the measured input voltage (vin), where the stimulus current (iin) is applied through the second and the fourth terminal, and the input voltage (vin) is measured between the second and the fourth terminal;
- determining a first corrected bodypart impedance Zwc as a parallel equivalent of the measured first bodypart impedance Zw and a stored first parasitic bodypart impedance (Zwp);
- determining a second corrected bodypart impedance Zfc as a parallel equivalent of the measured second bodypart impedance Zf and a stored second parasitic bodypart impedance (Zfp);
- determining an error impedance Zerr according to $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ wherein Zin is a stored input impedance; and
- determining a corrected body impedance (Zbcorr) as a difference between the measured body impedance (Zb) and the error impedance Zerr.

8. The method according to claim 7, further comprising
- determining a total impedance (Zt) in response to the stimulus current (iin) and the measured input voltage (vin), where the stimulus current (iin) is applied through the third and the fourth terminal, and the input voltage (vin) is measured between the third and the fourth terminal;
- determining a corrected total impedance (Ztc) as a parallel equivalent of the measured total impedance (Zt) and a stored parasitic total impedance (Ztp); and
- determining the corrected body impedance (Zbcorr) further based on the corrected total impedance (Ztc).

9. The method according to one of claims 7 or 8, wherein determining the stored first parasitic bodypart impedance (Zwp), the stored second parasitic bodypart impedance (Zfp) and the stored input impedance Zin is performed during a calibration phase.

10. The method according to claim 9, wherein during the calibration phase the first, second third and fourth terminals are connected to a calibration body via respective electrodes, and wherein the stored first parasitic bodypart impedance (Zwp), the stored second parasitic bodypart impedance (Zf) and the stored input impedance (Zin) are determined based on respective measurements and known impedance values of the calibration body corresponding to the measurements.

11. A computer program product comprising instructions which, when executed on one or more processors in connection with a first, a second, a third and a fourth terminal, cause the one or more processors to perform the bioimpedance measurement method according to one of claims 7 to 10.

## Patentansprüche

1. Bioimpedanz-Messschaltung, umfassend
- einen Satz von Anschlüssen, umfassend
- einen ersten Anschluss zum Anschließen einer ersten Elektrode (110), die am Körper anzubringen ist;
- einen zweiten Anschluss zum Anschließen einer zweiten Elektrode (120), die am Körper anzubringen ist;
- einen dritten Anschluss zum Anschließen einer dritten Elektrode (130), die am Körper anzubringen ist;
- einen vierten Anschluss zum Anschließen einer vierten Elektrode (140), die am Körper anzubringen ist;
- eine Steuerschaltung (200) zur Steuerung des Anlegens eines Stimulationsstroms (iin) mit einer Messfrequenz (F) über eine erste Teilmenge von zwei ausgewählten Anschlüssen des Satzes von Anschlüssen und zur Messung einer Eingangsspannung (vin) als Reaktion auf den Stimulationsstrom (iin) an einer zweiten Teilmenge von zwei ausgewählten Anschlüssen des Satzes von Anschlüssen;
- eine Auswerteschaltung (300) zur Ermittlung einer gemessenen Impedanz als Reaktion auf den Stimulationsstrom (iin) und die gemessene Eingangsspannung (vin); und
- eine Signalverarbeitungsschaltung (400) zur Ermittlung einer Körperimpedanz (Zb);
**dadurch gekennzeichnet, dass**
die Steuerschaltung (200) eingerichtet ist zur Steuerung der Messung
- einer Körperimpedanz (Zb), wobei der Stimulationsstrom (iin) über den ersten und den zweiten Anschluss angelegt wird und die Eingangsspannung (vin) zwischen dem dritten und dem vierten Anschluss gemessen wird;
- einer ersten Körperteilimpedanz (Zw), wobei der Stimulationsstrom (iin) über den ersten und den dritten Anschluss angelegt wird und die Eingangsspannung (vin) zwischen dem ersten und dem dritten Anschluss gemessen wird; und
- einer zweiten Körperteilimpedanz Zf, wobei der Stimulationsstrom (iin) über den zweiten und den vierten Anschluss angelegt wird und die Eingangsspannung (vin) zwischen dem zweiten und dem vierten Anschluss gemessen wird; und dass
die Signalverarbeitungsschaltung (400) eingerichtet ist, um
- eine erste korrigierte Körperteilimpedanz Zwc als Paralleläquivalent der gemessenen ersten Körperteilimpedanz Zw und einer gespeicherten ersten parasitären Körperteilimpedanz (Zwp) zu bestimmen;
- eine zweite korrigierte Körperteilimpedanz Zfc als Paralleläquivalent der gemessenen zweiten Körperteilimpedanz Zf und einer gespeicherten zweiten parasitären Körperteilimpedanz (Zfp) zu bestimmen;
- eine Fehlerimpedanz Zerr gemäß $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right)$ zu bestimmen, wobei Zin eine gespeicherte Eingangsimpedanz ist; und
- eine korrigierte Körperimpedanz (Zbcorr) als Differenz zwischen der gemessenen Körperimpedanz (Zb) und der Fehlerimpedanz Zerr zu bestimmen.

2. Bioimpedanz-Messschaltung nach Anspruch 1, wobei die Steuerschaltung (200) ferner eingerichtet ist, die Messung einer Gesamtimpedanz (Zt) zu steuern, wobei der Stimulationsstrom (iin) über den dritten und den vierten Anschluss angelegt wird und die Eingangsspannung (vin) zwischen dem dritten und dem vierten Anschluss gemessen wird, und wobei die Signalverarbeitungsschaltung (400) ferner eingerichtet ist, eine korrigierte Gesamtimpedanz (Ztc) als Paralleläquivalent der gemessenen Gesamtimpedanz (Zt) und einer gespeicherten parasitären Gesamtimpedanz (Ztp) zu ermitteln und die korrigierte Körperimpedanz (Zbcorr) ferner auf der Grundlage der korrigierten Gesamtimpedanz (Ztc) zu ermitteln.

3. Bioimpedanz-Messschaltung nach einem der Ansprüche 1 oder 2, wobei die Signalverarbeitungsschaltung (400) ferner eingerichtet ist, um die gespeicherte erste parasitäre Körperteilimpedanz Zwp, die gespeicherte zweite parasitäre Körperteilimpedanz Zf und die gespeicherte Eingangsimpedanz Zin während einer Kalibrierungsphase zu ermitteln.

4. Bioimpedanz-Messschaltung nach Anspruch 3, wobei während der Kalibrierungsphase der erste, der zweite, der dritte und der vierte Anschluss über jeweilige Elektroden mit einem Kalibrierkörper verbunden sind und wobei die Signalverarbeitungsschaltung (400) eingerichtet ist, die gespeicherte erste parasitäre Körperteilimpedanz (Zwp), die gespeicherte zweite parasitäre Körperteilimpedanz (Zfp) und die gespeicherte Eingangsimpedanz Zin auf der Grundlage entsprechender Messungen und bekannter Impedanzwerte des Kalibrierkörpers, die den Messungen entsprechen, zu ermitteln.

5. Bioimpedanz-Messschaltung nach einem der Ansprüche 1 bis 4, ferner umfassend eine Elektrodenanordnung (100) zum Anbringen an einem Körper, wobei die Elektrodenanordnung (100) die erste Elektrode (110), die zweite Elektrode (120), die dritte Elektrode (130) und die vierte Elektrode (140) umfasst, die jeweils mit dem entsprechenden Anschluss des Anschlusssatzes verbunden sind.

6. Elektronisches Gerät, das eine Bioimpedanz-Messschaltung gemäß einem der Ansprüche 1 bis 5 umfasst.

7. Verfahren zur Bioimpedanzmessung, das mit einem Satz von Anschlüssen durchgeführt wird, der umfasst:
- einen ersten Anschluss, der mit einer ersten Elektrode (110) verbunden ist, die an einem Körper angebracht ist;
- einen zweiten Anschluss, der mit einer zweiten Elektrode (120) verbunden ist, die an dem Körper angebracht ist;
- einen dritten Anschluss, der mit einer dritten Elektrode (130) verbunden ist, die an dem Körper angebracht ist; und
- einen vierten Anschluss, der mit einer vierten Elektrode (140) verbunden ist, die am Körper angebracht ist;
das Verfahren umfassend:
- Erzeugen eines Stimulationsstroms (iin) mit einer Messfrequenz (F);
**dadurch gekennzeichnet, dass** das Verfahren ferner umfasst:
- Bestimmen einer Körperimpedanz (Zb) in Reaktion auf den Stimulationsstrom (iin) und einer gemessenen Eingangsspannung (vin) als Reaktion auf den Stimulationsstrom (iin), wobei der Stimulationsstrom (iin) über den ersten und den zweiten Anschluss angelegt wird und die Eingangsspannung (vin) zwischen dem dritten und dem vierten Anschluss gemessen wird;
- Ermitteln einer ersten Körperteilimpedanz Zw als Reaktion auf den Stimulationsstrom (iin) und die gemessene Eingangsspannung (vin), wobei der Stimulationsstrom (iin) über den ersten und den dritten Anschluss angelegt wird und die Eingangsspannung (vin) zwischen dem ersten und dem dritten Anschluss gemessen wird;
- Bestimmen einer zweiten Körperteilimpedanz Zf in Abhängigkeit vom Stimulationsstrom (iin) und der gemessenen Eingangsspannung (vin), wobei der Stimulationsstrom (iin) über den zweiten und den vierten Anschluss angelegt wird und die Eingangsspannung (vin) zwischen dem zweiten und dem vierten Anschluss gemessen wird;
- Bestimmen einer ersten korrigierten Körperteilimpedanz Zwc als Paralleläquivalent der gemessenen ersten Körperteilimpedanz Zw und einer gespeicherten ersten parasitären Körperteilimpedanz (Zwp);
- Bestimmen einer zweiten korrigierten Körperteilimpedanz Zfc als Paralleläquivalent der gemessenen zweiten Körperteilimpedanz Zf und einer gespeicherten zweiten parasitären Körperteilimpedanz (Zfp);
- Bestimmen einer Fehlerimpedanz Zerr gemäß $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ wobei Zin eine gespeicherte Eingangsimpedanz ist; und
- Bestimmen einer korrigierten Körperimpedanz (Zbcorr) als Differenz zwischen der gemessenen Körperimpedanz (Zb) und der Fehlerimpedanz Zerr.

8. Verfahren nach Anspruch 7, das ferner umfasst:
- Bestimmen einer Gesamtimpedanz (Zt) in Abhängigkeit vom Stimulationsstrom (iin) und der gemessenen Eingangsspannung (vin), wobei der Stimulationsstrom (iin) über den dritten und den vierten Anschluss angelegt wird und die Eingangsspannung (vin) zwischen dem dritten und dem vierten Anschluss gemessen wird;
- Ermitteln einer korrigierten Gesamtimpedanz (Ztc) als Paralleläquivalent der gemessenen Gesamtimpedanz (Zt) und einer gespeicherten parasitären Gesamtimpedanz (Ztp); und
- Ermitteln der korrigierten Körperimpedanz (Zbcorr) ferner auf der Grundlage der korrigierten Gesamtimpedanz (Ztc).

9. Verfahren nach einem der Ansprüche 7 oder 8, wobei das Bestimmen der gespeicherten ersten parasitären Körperteilimpedanz (Zwp), der gespeicherten zweiten parasitären Körperteilimpedanz (Zfp) und der gespeicherten Eingangsimpedanz (Zin) während einer Kalibrierungsphase erfolgt.

10. Verfahren nach Anspruch 9, wobei während der Kalibrierungsphase der erste, der zweite, der dritte und der vierte Anschluss über jeweilige Elektroden mit einem Kalibrierkörper verbunden werden und wobei die gespeicherte erste parasitäre Körperteilimpedanz (Zwp), die gespeicherte zweite parasitäre Körperteilimpedanz (Zf) und die gespeicherte Eingangsimpedanz (Zin) auf der Grundlage jeweiliger Messungen und bekannter Impedanzwerte des Kalibrierkörpers, die den Messungen entsprechen, ermittelt werden.

11. Computerprogrammprodukt, das Befehle umfasst, die, wenn sie auf einem oder mehreren Prozessoren in Verbindung mit einem ersten, einem zweiten, einem dritten und einem vierten Anschluss ausgeführt werden, bewirken, dass der eine oder die mehreren Prozessoren das Verfahren zur Bioimpedanzmessung gemäß einem der Ansprüche 7 bis 10 durchführen.

## Revendications

1. Circuit de mesure de bioimpédance, comprenant
- un ensemble de bornes comprenant
- une première borne destinée à connecter une première électrode (110) à appliquer sur un corps ;
- une deuxième borne destinée à connecter une deuxième électrode (120) à appliquer sur le corps ;
- une troisième borne destinée à connecter une troisième électrode (130) à appliquer sur le corps ;
- une quatrième borne destinée à connecter une quatrième électrode (140) à appliquer sur le corps ;
- un circuit de commande (200) destiné à commander l'application d'un courant de stimulation (iin) à une fréquence de mesure (F) par l'intermédiaire d'un premier sous-ensemble de deux bornes sélectionnées parmi l'ensemble de bornes, et la mesure d'une tension d'entrée (vin) en réponse au courant de stimulation (iin) au niveau d'un deuxième sous-ensemble de deux bornes sélectionnées parmi l'ensemble de bornes ;
- un circuit d'évaluation (300) destiné à déterminer une impédance mesurée en réponse au courant de stimulation (iin) et à la tension d'entrée mesurée (vin) ; et
- un circuit de traitement de signal (400) destiné à déterminer une impédance corporelle (Zb) ;
**caractérisé en ce que**
le circuit de commande (200) est configuré pour commander la mesure de
- une impédance corporelle (Zb), le courant de stimulation (iin) étant appliqué entre la première et la deuxième borne, et la tension d'entrée (vin) étant mesurée entre la troisième et la quatrième borne ;
- une première impédance de partie du corps (Zw), le courant de stimulation (iin) étant appliqué entre la première et la troisième borne, et la tension d'entrée (vin) étant mesurée entre la première et la troisième borne ; et
- une deuxième impédance de partie du corps Zf, où le courant de stimulation (iin) est appliqué entre la deuxième et la quatrième borne, et la tension d'entrée (vin) est mesurée entre la deuxième et la quatrième borne ; et **en ce que**
le circuit de traitement du signal (400) est configuré pour
- déterminer une première impédance corrigée de partie du corps Zwc en tant qu'équivalent en parallèle de la première impédance de partie du corps mesurée Zw et d'une première impédance parasite de partie du corps mémorisée (Zwp) ;
- déterminer une deuxième impédance corrigée de partie du corps Zfc en tant qu'équivalent en parallèle de la deuxième impédance de partie du corps mesurée Zf et d'une deuxième impédance parasite de partie du corps mémorisée (Zfp) ;
- déterminer une impédance d'erreur Zerr selon la formule $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ où Zin est une impédance d'entrée mémorisée ; et
- déterminer une impédance corporelle corrigée (Zbcorr) comme étant la différence entre l'impédance corporelle mesurée (Zb) et l'impédance d'erreur Zerr.

2. Circuit de mesure de bioimpédance selon la revendication 1, dans lequel le circuit de commande (200) est en outre configuré pour commander la mesure d'une impédance totale (Zt), le courant de stimulation (iin) étant appliqué par l'intermédiaire des troisième et quatrième bornes, et la tension d'entrée (vin) étant mesurée entre les troisième et quatrième bornes, et dans lequel le circuit de traitement de signal (400) est en outre configuré pour déterminer une impédance totale corrigée (Ztc) en tant qu'équivalent en parallèle de l'impédance totale mesurée (Zt) et d'une impédance totale parasite mémorisée (Ztp), et à déterminer l'impédance corporelle corrigée (Zbcorr) en se basant en outre sur l'impédance totale corrigée (Ztc).

3. Circuit de mesure de bioimpédance selon l'une des revendications 1 ou 2, dans lequel le circuit de traitement du signal (400) est en outre configuré pour déterminer la première impédance parasite de partie du corps mémorisée (Zwp), la deuxième impédance parasite de partie du corps mémorisée (Zf) et l'impédance d'entrée mémorisée (Zin) au cours d'une phase d'étalonnage.

4. Circuit de mesure de bioimpédance selon la revendication 3, dans lequel, pendant la phase d'étalonnage, les première, deuxième, troisième et quatrième bornes sont connectées à un corps d'étalonnage via des électrodes respectives, et dans lequel le circuit de traitement de signal (400) est configuré pour déterminer la première impédance parasite de partie du corps stockée (Zwp), la deuxième impédance parasite de partie du corps enregistrée (Zfp) et l'impédance d'entrée enregistrée Zin sur la base de mesures respectives et de valeurs d'impédance connues du corps d'étalonnage correspondant aux mesures.

5. Circuit de mesure de bioimpédance selon l'une des revendications 1 à 4, comprenant en outre un agencement d'électrodes (100) destiné à être fixé sur un corps, l'agencement d'électrodes (100) comprenant la première électrode (110), la deuxième électrode (120), la troisième électrode (130) et la quatrième électrode (140) respectivement connectées à la borne correspondante de l'ensemble de bornes.

6. Dispositif électronique comprenant un circuit de mesure de bioimpédance selon l'une des revendications 1 à 5.

7. Procédé de mesure de bioimpédance mis en œuvre à l'aide d'un ensemble de bornes comprenant
- une première borne reliée à une première électrode (110) fixée au corps ;
- une deuxième borne reliée à une deuxième électrode (120) fixée au corps ;
- une troisième borne reliée à une troisième électrode (130) fixée au corps ; et
- une quatrième borne reliée à une quatrième électrode (140) fixée au corps ;
le procédé comprenant :
- générer un courant de stimulation (iin) à une fréquence de mesure (F) ;
**caractérisé en ce que** le procédé comprend en outre :
- déterminer une impédance corporelle (Zb) en réponse au courant de stimulation (iin) et à une tension d'entrée mesurée (vin) en réponse au courant de stimulation (iin), le courant de stimulation (iin) étant appliqué par l'intermédiaire des première et deuxième bornes, et la tension d'entrée (vin) étant mesurée entre les troisième et quatrième bornes ;
- déterminer une première impédance de partie du corps (Zw) en réponse au courant de stimulation (iin) et à la tension d'entrée mesurée (vin), le courant de stimulation (iin) étant appliqué entre la première et la troisième borne, et la tension d'entrée (vin) étant mesurée entre la première et la troisième borne ;
- déterminer une deuxième impédance de partie du corps Zf en réponse au courant de stimulation (iin) et à la tension d'entrée mesurée (vin), le courant de stimulation (iin) étant appliqué entre la deuxième et la quatrième borne, et la tension d'entrée (vin) étant mesurée entre la deuxième et la quatrième borne ;
- déterminer une première impédance corrigée de partie du corps Zwc en tant qu'équivalent en parallèle de la première impédance mesurée de partie du corps Zw et d'une première impédance parasite de partie du corps (Zwp) mémorisée ;
- déterminer une deuxième impédance corrigée de partie du corps Zfc en tant qu'équivalent en parallèle de la deuxième impédance mesurée de partie du corps Zf et d'une deuxième impédance parasite de partie du corps (Zfp) mémorisée ;
- déterminer une impédance d'erreur Zerr selon la formule $Zerr = \frac{1}{2} ⋅ \left(\frac{Zin ⋅ {\left(Zfc-Zwc\right)}^{2}}{\left(Zwc+Zin\right) ⋅ \left(Zfc+Zin\right)}\right) ,$ où Zin est une impédance d'entrée mémorisée ; et
- déterminer une impédance corporelle corrigée (Zbcorr) comme étant la différence entre l'impédance corporelle mesurée (Zb) et l'impédance d'erreur Zerr.

8. Procédé selon la revendication 7, comprenant en outre
- déterminer une impédance totale (Zt) en réponse au courant de stimulation (iin) et à la tension d'entrée mesurée (vin), le courant de stimulation (iin) étant appliqué entre la troisième et la quatrième borne, et la tension d'entrée (vin) étant mesurée entre la troisième et la quatrième borne ;
- déterminer une impédance totale corrigée (Ztc) comme équivalent en parallèle de l'impédance totale mesurée (Zt) et d'une impédance totale parasite mémorisée (Ztp) ; et
- déterminer l'impédance corporelle corrigée (Zbcorr) en se basant en outre sur l'impédance totale corrigée (Ztc).

9. Procédé selon l'une des revendications 7 ou 8, dans lequel la détermination de la première impédance parasite de partie du corps mémorisée (Zwp), de la deuxième impédance parasite de partie du corps mémorisée (Zfp) et de l'impédance d'entrée mémorisée (Zin) est effectuée au cours d'une phase d'étalonnage.

10. Procédé selon la revendication 9, dans lequel, au cours de la phase d'étalonnage, les première, deuxième, troisième et quatrième bornes sont connectées à un corps d'étalonnage via des électrodes respectives, et dans lequel la première impédance parasite de partie du corps mémorisée (Zwp), la deuxième impédance parasite de partie du corps mémorisée (Zf) et l'impédance d'entrée mémorisée (Zin) sont déterminées sur la base de mesures respectives et de valeurs d'impédance connues du corps d'étalonnage correspondant aux mesures.

11. Produit logiciel comprenant des instructions qui, lorsqu'elles sont exécutées sur un ou plusieurs processeurs en liaison avec une première, une deuxième, une troisième et une quatrième borne, amènent le ou les processeurs à mettre en œuvre le procédé de mesure de bioimpédance selon l'une des revendications 7 à 10.
